# EUROPÄISCHE PATENTSCHRIFT

(11) **EP 4 057 952 B1**
(45) Veröffentlichungstag und Bekanntmachung des Hinweises auf die Patenterteilung: **30.07.2025**
(21) Anmeldenummer: 20801245.0
(22) Anmeldetag: 04.11.2020
(51) Int. Cl.: A61F 5/02

(54) **ORTHOPÄDIETECHNISCHE EINRICHTUNG**
ORTHOPAEDIC DEVICE
DISPOSITIF ORTHOPÉDIQUE

(30) Priorität: 11.11.2019 DE 102019130391
(43) Veröffentlichungstag der Anmeldung: 21.09.2022
(73) Patentinhaber: Ottobock SE & Co. KGaA, 37115 Duderstadt (DE)
(72) Erfinder: MIZERA, Oliver, 37083 Göttingen (DE); VOGEL, Carsten, 37115 Duderstadt (DE); TÜTTEMANN, Markus, 45731 Waltrop (DE)
(74) Vertreter: Gramm, Lins & Partner Patent- und Rechtsanwälte PartGmbB
(86) Internationale Anmeldenummer: PCT/EP2020/080909
(87) Internationale Veröffentlichungsnummer: WO 2021/094158

(56) Entgegenhaltungen:
- DE-A1- 102019 119 645
- US-A1- 2017 196 712
- US-A1- 2017 360 588

## Beschreibung

Die Erfindung betrifft eine orthopädietechnische Einrichtung, die ein Gelenk mit einem ersten Gelenkelement, das einen ersten Gelenkarm mit einem ersten Formschlusselement aufweist, und einem relativ zu dem ersten Gelenkelement verschwenkbaren zweiten Gelenkelement, das einen zweiten Gelenkarm und einen Kraftangriffshebel mit einem zweiten Formschlusselement und einen mechanischen Energiespeicher aufweist, der zwischen dem Kraftangriffshebel und dem zweiten Gelenkarm angeordnet ist, wobei der mechanische Energiespeicher aufladbar und entladbar ist, indem der erste Gelenkarm relativ zu dem zweiten Gelenkarm verschwenkt wird, wenn das erste Formschlusselement mit dem zweiten Formschlusselement in Eingriff ist.

Eine derartige Vorrichtung ist beispielsweise aus der nicht vorveröffentlichten DE 10 2019 119 645 bekannt. Es handelt sich um eine orthopädietechnische Einrichtung zum Unterstützen eines unteren Rückens eines Benutzers. Derartige Vorrichtungen sind aus dem Stand der Technik seit Langem bekannt und werden insbesondere beim Heben verwendet, um die Person, die beispielsweise einen schweren Gegenstand heben soll, zu unterstützen. Zusätzlich werden derartige Vorrichtungen für Personen verwendet, die in gebückter Haltung arbeiten müssen.

Eine derartige Vorrichtung ist beispielsweise aus der US 443,113 bekannt. Sie verfügt über Oberschenkelelemente, die an den Oberschenkeln des Trägers angeordnet werden. Über Schultergurte wird die Vorrichtung auch am Oberkörper des Trägers angeordnet. Zwischen den Schultergurten und den Oberschenkelelementen befinden sich Blattfederelemente, die beim Bücken gebogen und damit mit potentieller Energie aufgeladen werden. Dadurch üben die Blattfederelemente eine Kraft auf den Oberkörper aus, der die Streckung des Körpers unterstützt. Nachteilig ist jedoch, dass die entstehende Kraft immer ausgeübt wird, wenn ein Winkel zwischen dem Oberkörper und den Oberschenkeln verändert wird. Sie wird folglich beispielsweise auch beim Treppensteigen oder Sitzen ausgeübt, was zumindest unbequem, gegebenenfalls jedoch auch störend und unbequem ist.

Prinzipiell ähnliche Vorrichtungen sind beispielsweise aus der US 2017/0196712 A1 und der US 2017/0360588 A1 bekannt. Die den unteren Rücken oder den Oberkörper unterstützende Kraft, die das Aufrichten der Person erleichtern soll, wird jedoch nicht immer ausgeübt. Im erstgenannten Stand der Technik kommt es erst bei Überschreiten eines gewissen Neigungswinkels, wenn also der Winkel zwischen dem Oberkörperelement der Vorrichtung und dem Oberschenkelelement der Vorrichtung einen vorbestimmten Winkel unterschreitet, zur Ausübung der Kraft. Bis zu diesem Winkel kann der Oberkörper relativ zum Oberschenkel geneigt werden, ohne dass ein Aktuator oder Energiespeicher energetisch aufgeladen wird. Dennoch kommt es bei dieser Vorrichtung immer dann zu einer unterstützenden Kraft, wenn der Oberkörper relativ zum Oberschenkel einen Winkel einschließt, der kleiner als der vorbestimmte Grenzwinkel ist, also der Oberkörper relativ zum Oberschenkel geneigt wird.

Aus dem letztgenannten Stand der Technik ist eine Vorrichtung bekannt, bei der die unterstützende Kraft immer dann ausgeübt wird, wenn der Oberkörper relativ zur Vertikalen, also der Richtung, entlang derer die Gewichtskraft wirkt, einen vorbestimmten Winkel einschließt. Damit wird verhindert, dass die Kraft beispielsweise ausgeübt wird, wenn der Träger der Vorrichtung sitzt, sofern dabei der Oberkörper nicht den vorbestimmten Winkel zur Vertikalen überschreitet. Neigt die Person jedoch beim Sitzen den Oberkörper soweit, dass der vorbestimmte Winkel überschritten wird, wird automatisch eine unterstützende Kraft ausgeübt. Nachteilig bei allen genannten Richtungen ist, dass nicht sicher eingestellt und erreicht werden kann, dass die Kraft nur dann ausgeübt wird, wenn sie benötigt und gewünscht ist, sondern auch Situationen und Bewegungen auftreten können, bei denen keine unterstützende Kraft benötigt wird oder der untere Rücken nicht beansprucht oder unterstützt werden muss. Dieses Problem wurde mit der Vorrichtung aus der bereits genannten DE 102019 119 645 behoben.

Das Problem wurde gelöst, indem der mechanische Energiespeicher nur dann aufladbar und entladbar ist, wenn die beiden Zahnräder in Eingriff miteinander stehen. Dies geschieht, wenn ein bestimmtes Kriterium erfüllt ist. Im genannten Ausführungsbeispiel werden die beiden Zahnräder in Eingriff miteinander gebracht, wenn der Oberkörper des Trägers der Vorrichtung einen bestimmten Winkel zum Beckenbereich überschreitet. Nachteilig ist jedoch, dass sich bis zu diesem Zeitpunkt die beiden Zahnräder relativ zueinander bewegen können, sodass nicht im Vorhinein klar ist, wie die Zähne der Zahnräder zueinander orientiert sind, wenn die Zahnräder in Eingriff gebracht werden sollen. Es kann daher passieren, dass die Zähne nur in einem sehr kleinen Bereich, nämlich den Zahnspitzen, miteinander in Eingriff stehen. Dies ist insbesondere bei größeren zu übertragenden Kräften kein sicherer Formschluss, sodass die Gefahr besteht, dass die beiden Zahnräder aneinander abrutschen und unkontrolliert die im mechanischen Energiespeicher gespeicherte Energie abgegeben wird.

Der Erfindung liegt daher die Aufgabe zugrunde, eine orthopädietechnische Einrichtung der oben genannten Art so weiterzuentwickeln, dass dieses Problem behoben oder zumindest gemildert wird.

Die Erfindung löst die gestellte Aufgabe durch eine orthopädietechnische Einrichtung gemäß dem Oberbegriff des Anspruchs 1, die sich dadurch auszeichnet, dass sie eine Sicherungseinrichtung aufweist, durch die sichergestellt ist, dass unabhängig von der Position des ersten Formschlusselementes und des zweiten Formschlusselementes relativ zueinander die beiden Formschlusselemente derart in Eingriff bringbar sind, dass eine von dem aufgeladenen mechanischen Energiespeicher wirkende Kraft von dem zweiten Formschlusselement auf das erste Formschlusselement übertragen wird. Dadurch wird sichergestellt, dass das unkontrollierte Abgeben der Energie beim Abrutschen der Formschlusselemente aneinander, verhindert wird.

Die Formschlusselemente verfügen über Vertiefungen und/oder Vorsprünge, die so ausgebildet sind, dass die beiden Formschlusselemente formschlüssig miteinander in Eingriff bringbar sind. Bevorzugt handelt es sich um Zahnräder, besonders bevorzugt um Stirnzahnräder.

Prinzipiell sind unterschiedliche Mechanismen denkbar, mit denen die Sicherungseinrichtung die gestellte Aufgabe löst. Vorzugsweise ist die Sicherungseinrichtung eingerichtet, beim in Eingriff Bringen oder nachdem in Eingriff Bringen der beiden Formschlusselemente die Formschlusselemente relativ zueinander zu drehen. Sollten sich die Formschlusselemente zu dem Zeitpunkt, zudem sie miteinander in Eingriff gebracht werden sollen, so zueinander positioniert haben, dass die Vorsprünge und/oder Vertiefungen der beiden Formschlusselemente nicht vollständig, sondern nur in einem kleinen Bereich miteinander in Eingriff gebracht werden können, kann durch die Drehung der beiden Formschlusselemente relativ zueinander diese Relativposition verändert und so ein vollständiger oder zumindest größere Eingriff sichergestellt werden.

Vorzugsweise weisen das erste Formschlusselement und das zweite Formschlusselement stirnseitige Vorsprünge und/oder Vertiefungen auf. Dies ist beispielsweise bei Stirnzahnrädern gegeben.. Unter einem Stirnzahnrad wird dabei ein Zahnrad verstanden, dessen Zähne in axialer Richtung hervorstehenden. Bei einem herkömmlichen Zahnrad sind die Zähne am äußeren Umfang des Zahnrades angeordnet und tragen in radialer Richtung hervor. Ein Zahnrad verfügt über eine Rotationsachse, um die es drehbar gelagert ist und bezüglich derer die Begriffe axial und radial zu verstehen sind. Bei einem Stirnzahnrad hingegen befinden sich die Zähne an einer Stirnfläche des Zahnrades und ragen somit in axialer Richtung hervor. Werden zwei derartige Stirnzahnräder miteinander in Eingriff gebracht, greifen vorzugsweise alle Zähne des einen Zahnrades in die Zähne des anderen Zahnrades, sodass der Kontaktbereich deutlich größer ist als dies bei herkömmlichen Zahnrädern, deren Zähne am äußeren Umfang angeordnet sind, ist. Dadurch kann eine größere Kraft übertragen werden.

Werden zwei derartige Stirnzahnräder in Eingriff gebracht, deren Zähne nicht optimal zueinander positioniert sind, kann dies folglich korrigiert werden, indem die beiden Zahnräder relativ zueinander gedreht werden. Die benötigte Drehung ist dabei vorzugsweise klein, insbesondere kleiner als 5°, bevorzugt kleiner als 3°, besonders bevorzugt kleiner als 2°. Dies ist auch bei Formschlusselementen der Fall, die keine Zahnräder mit Zähnen sind, sondern andere Vertiefungen und/oder Vorsprünge aufweisen.

Besonders bevorzugt weist die Sicherungseinrichtung eine aus einem der Formschlusselemente axial hervorstehende Führungsspindel auf, die stirnseitige Vertiefungen und/oder Vorsprünge, insbesondere eine Stirnverzahnung aufweist, die eingerichtet ist, mit dem jeweils anderen Formschlusselement zusammenzuwirken.

Vorteilhafterweise ist die Führungsspindel in axialer Richtung relativ zu dem Formschlusselement, aus dem sie axial hervorstehende, verschiebbar, wobei die Führungsspindel derart ausgebildet ist, das beim axialen Verschieben die Führungsspindel um ihre Längsachse gedreht wird, sodass ein Drehmoment auf das Formschlusselement ausgeübt wird, das mit den stirnseitigen Vorsprüngen und/oder Vertiefungen der Führungsspindel zusammenwirkt. Bei einer derartigen Ausgestaltung steht die Führungsspindel axial aus der Stirnfläche eines der beiden Formschlusselemente hervor, wenn die beiden Formschlusselemente nicht in Eingriff miteinander sind. Sollen die beiden Formschlusselemente nun in Eingriff gebracht werden, wird eines der beiden Formschlusselemente, vorzugsweise das, aus dem keine Führungsspindel hervor steht, in Richtung auf das jeweils andere Formschlusselement bewegt. Dabei kommen zunächst die stirnseitigen Vorsprünge und/oder Vertiefungen, insbesondere die Stirnverzahnung der Führungsspindel mit den stirnseitigen Vertiefungen und/oder Vorsprüngen des anderen Formschlusselementes in Kontakt. Dadurch wird jedoch die Bewegung des anderen Formschlusselementes auf das mit der Führungsspindel versehene Formschlusselement nicht gestoppt, sondern die Führungsspindel wird in axialer Richtung verschoben und in das Formschlusselement, an dem sie angeordnet ist, hineinbewegt.

Vorzugsweise bilden die stirnseitigen Vorsprünge und/oder Vertiefungen, insbesondere die Stirnverzahnung der Führungsspindel mit den stirnseitigen Vertiefungen und/oder Vorsprüngen, insbesondere der Stirnverzahnung ihres Formschlusselement eine durchgehende Verzahnung, wenn die Führungsspindel soweit in ihr Zahnrad verschoben ist, dass sich eine einzige durchgehende Stirnfläche bildet.

Greifen die Vorsprünge und/oder Vertiefungen des anderen Formschlusselementes, das auf die Führungsspindel zu verschoben wurde, nicht optimal in deren Vorsprünge und/oder Vertiefungen ein, über die Führungsspindel ein Drehmoment auf dieses Formschlusselement aus, sodass es in die optimale Position gedreht wird, um in die Vertiefungen und/oder Vorsprünge des anderen Formschlusselementes einzugreifen. Dabei wird diese optimale Position vorzugsweise dann erreicht, wenn die Führungsspindel vollständig in ihrem Formschlusselement versenkt ist. Auf diese Weise wird sichergestellt, dass die Vorsprünge und/oder Vertiefungen der beiden Formschlusselemente in optimaler Position zueinander ineinander eingreifen, unabhängig davon, in welcher Position die beiden Formschlusselemente relativ stehen, wenn sie miteinander in Eingriff gebracht werden sollen.

Alternativ oder zusätzlich dazu weist das erste Formschlusselement und/oder das zweite Formschlusselement wenigstens zwei Teilformschlusselemente, beispielsweise wenigstens zwei Teilzahnräder auf, die in axialer Richtung unabhängig voneinander bewegbar sind. Werden die beiden Formschlusselement in dieser Ausgestaltung aufeinander zu bewegt, um sie miteinander in Eingriff zu bringen, greift eines der Teilformschlusselemente des einen Formschlusselement in die Vorsprünge und/oder Vertiefungen des anderen Formschlusselement ein, bevor die anderen Teilformschlusselemente dies tun. Die Teilformschlusselement sind vorzugsweise in Umfangsrichtung versetzt zueinander angeordnet, sodass die Vorsprünge und/oder Vertiefungen, insbesondere Zähne jedes einzelnen der Teilformschlusselement untereinander zwar äquidistant angeordnet sind, zwischen den Vorsprüngen und/oder Vertiefungen, insbesondere Zähnen benachbarter Teilformschlusselemente jedoch ein Winkelversatz vorhanden ist. Auf diese Weise ist sichergestellt, dass die Vorsprünge und/oder Vertiefungen unterschiedlicher Teilformschlusselemente unterschiedlich gut in die Vorsprünge und/oder Vertiefungen des jeweils anderen Formschlusselementes eingreifen, wenn die beiden Formschlusselemente miteinander in Eingriff gebracht werden.

Greifen die Vorsprünge und/oder Vertiefungen des ersten Teilformschlusselementes folglich optimal in die Vorsprünge und/oder Vertiefungen des anderen Formschlusselementes ein, ist es ausreichend, um die aufzubringenden Kräfte zu übertragen. Ist dies jedoch nicht der Fall, da die Vorsprünge und/oder Vertiefungen des ersten Teilformschlusselements beispielsweise nur im Bereich der Spitzen mit den Vertiefungen und/oder Vorsprüngen des anderen Formschlusselementes in Eingriff stehen, greifen die Vorsprünge und/oder Vertiefungen eines der weiteren Teilformschlusselementes besser in das andere Formschlusselement ein. Ist der Kontakt zwischen den Spitzen der Vorsprünge und/oder Vertiefungen des ersten Teilformschlusselementes mit den Spitzen der Vorsprünge und/oder Vertiefungen des anderen Formschlusselementes nicht ausreichend, um die auftretenden Kräfte sicher zu übertragen, kommt es zu einem "Durchrutschen" der beiden Formschlusselemente. Dies wird jedoch bereits nach einer Relativbewegung von wenigen Grad durch die Vorsprünge und/oder Vertiefungen eines der weiteren Teilformschlusselemente abgefangen, die aufgrund des Winkelversatzes zwischen den Vorsprüngen und/oder Vertiefungen unterschiedlicher Teilformschlusselemente besser in die Vorsprünge und/oder Vertiefungen des anderen Formschlusselementes eingreifen.

Vorzugsweise weisen daher die wenigstens zwei Teilformschlusselemente die gleichen Vorsprünge und/oder Vertiefungen, insbesondere die gleiche Zählung auf, die jedoch in Umfangsrichtung gegeneinander versetzt sind. Vorzugsweise ist der Versatz kleiner als 10°, bevorzugt kleiner als 7°, besonders bevorzugt kleiner als 5°. Die gleiche Zähnung bedeutet in diesem Zusammenhang, dass die Zähne die gleiche Tiefe, den gleichen Flankenverlauf, und den gleichen Winkelversatz zueinander aufweisen.

In einer bevorzugten Ausgestaltung sind die wenigstens zwei Teilformschlusselemente in axialer Richtung voneinander beabstandet, wenn das erste Formschlusselement und das zweite Formschlusselement nicht miteinander in Eingriff stehen. Auf diese Weise ist sichergestellt, welches der wenigstens zwei Teilformschlusselemente das erste Teilformschlusselement ist, das mit anderen Formschlusselement in Kontakt kommen.

Ein Teilformschlusselement ist dabei wie ein Tortenstück geformt. Es verfügt bevorzugt über zwei gerade Kanten und eine kreisbogenförmige Kante. Es handelt sich bevorzugt um ein Kreissegment. An der Stirnfläche befindet sich vorzugsweise die ebenfalls kreissegmentförmige Zähnung.

Vorzugsweise ist das erste Gelenkelement ein Oberkörperelement und das zweite Gelenkelement ein Oberschenkelelement. Die Einrichtung verfügt zudem über ein Beckenelement, wobei die beiden Formschlusselemente in Eingriff und außer Eingriff bringbar sind, in dem das Oberkörperelement relativ zu dem Beckenelement bewegt wird. Dieser Ausgestaltung der Erfindung liegt die Erkenntnis zugrunde, dass der untere Rücken nicht immer dann einer Unterstützung bedarf, wenn ein Winkel zwischen einem Oberkörperelement, das beispielsweise im Brustbereich oder im Rückenbereich des Oberkörpers des Trägers angeordnet wird, und dem Oberschenkel des Trägers einen vorbestimmten Winkel unterschreitet, die beiden Körperteile also gegeneinander verschwenkt werden. Vielmehr ist eine Unterstützung nur dann notwendig, wenn es zu einer Verschwenkung zwischen dem Oberkörper, also beispielsweise den Brustkorb, des Trägers und dem Becken des Trägers kommt. Durch die Ausgestaltung der Vorrichtung in dieser Weise wird erreicht, dass immer dann, wenn diese Verschwenkung zwischen dem Oberkörper und dem Becken des Trägers eintritt, eine unterstützende Kraft ausgeübt wird. Wird hingegen der Oberkörper relativ zum Oberschenkel verschwenkt, ohne dass es zu einer Bewegung des Oberkörpers relativ zum Becken kommt, soll keine Kraft ausgeübt werden. **In** diesem Fall werden die beiden Zahnräder nicht in Eingriff miteinander gebracht.

Bevorzugt sind an dem Beckenelement oder an dem Oberschenkelelement wenigstens zwei Magnete und an dem jeweils anderen Element wenigstens ein Magnet derart angeordnet, dass sie eine Kraft aufeinander ausüben, deren Richtung wechselt, wenn der Winkel beim Bewegen des Oberkörperelementes relativ zu dem Beckenelement den vorbestimmten Grenzwinkel passiert. **In** dieser Ausgestaltung verfügt die Verschiebeeinrichtung folglich über die genannten Magnete. An dem Element am Oberkörperelement oder am Beckenelement, an dem wenigstens zwei Magnete angeordnet sind, sind diese bevorzugt in unterschiedlicher Orientierung angeordnet. Dies bedeutet, dass bei wenigstens einem der Magnete der Nordpol und bei wenigstens einem anderen der Südpol in Richtung auf das jeweils andere Element der orthopädietechnischen Einrichtung gerichtet ist.

Ist der Winkel zwischen dem Oberkörperelement und dem Beckenelement größer als der vorbestimmte Grenzwinkel sind die beiden Formschlusselemente nicht miteinander in Eingriff. Bevorzugt wird folglich durch die Magnete eine Kraft ausgeübt, die die beiden Formschlusselemente voneinander weg hält. Dies kann geschehen, indem die Magnete eine Kraft aufeinander ausüben. Dies kann beispielsweise eine abstoßende Kraft sein. Dies wird erreicht, indem ein Magnet des Beckenelementes und ein Magnet des Oberschenkelelementes nahe aneinander positioniert sind, sodass ihre gleichnamigen Pole, also jeweils Südpol oder jeweils Nordpol, aufeinander zu gerichtet sind. Wird nun das Beckenelement relativ zum Oberschenkelelement bewegt, werden auch die an den jeweiligen Elementen angeordneten Magnete bewegt. Es kommt folglich zu einer Verschiebung der bewegten Magnete zueinander. **In** dem Moment, in dem der Winkel des Oberkörperelementes relativ zum Beckenelement den vorbestimmten Grenzwinkel passiert, kommt vorzugsweise ein zweiter Magnet des Beckenelementes oder des Oberschenkelelementes in den Bereich des wenigstens einen Magnetes des jeweils anderen Elementes. Dabei kommt es nun zu einer anziehenden Kraft, da ungleichnamige Pole der beiden Magnete zueinander gerichtet werden.

Vorzugsweise verfügen zumindest einige, besonders bevorzugt jedoch alle, Vorsprünge und/oder Vertiefungen eines der Formschlusselemente, bevorzugt jedoch beider Formschlusselemente, über eine Hinterschnitt-Verzahnung. Dies bedeutet, dass vorzugsweise beide Flanken einer Vertiefung und/oder eines Vorsprunges in die gleiche Richtung geneigt sind. Dadurch kann allein durch die übertragenen Kräfte ein Drehmoment auf eines der Formschlusselemente ausgeübt werden, das in eine Kraft überführt wird, die eine axiale Komponente hat. Dadurch werden die beiden Formschlusselemente näher aufeinander zu gezogen, sodass die Stärke der Verzahnung, also des Eingriffs der beiden Formschlusselemente ineinander, erhöht wird.

Vorzugsweise sind die Formschlusselemente miteinander in Eingriff bringbar, in dem eines der Formschlusselemente auf das andere Formschlusselement zu bewegt wird, das relativ zu dem Bauteil, an dem es angeordnet ist, in einer Richtung drehbar gelagert ist. Dabei handelt es sich bevorzugt um eine schwimmende Lagerung, die eine geringfügige Rotation um beispielsweise weniger als 15°, bevorzugt weniger als 10°, besonders bevorzugt weniger als 5°, ermöglicht und so sicherstellt, dass die optimale Position und Orientierung der beiden Formschlusselemente zueinander erreicht werden kann.

Vorzugsweise weist die orthopädietechnische Einrichtung ein Oberkörperelement, ein Oberschenkelelement und einen ersten passiven Aktuator auf, der eingerichtet ist, eine Kraft auf das Oberschenkelelement und/oder das Oberkörperelement auszuüben, wenn ein Winkel, der zwischen dem Oberschenkelelement und dem Oberkörperelement eingeschlossen ist, in einem ersten vorbestimmten Winkelbereich lieg. Besonders bevorzugt weist die Einrichtung wenigstens einen zweiten passiven Aktuator auf, der eingerichtet ist, eine Kraft auf das Oberschenkelelement und/oder das Oberkörperelement auszuüben, wenn der Winkel in einem vorbestimmten zweiten Winkelbereich liegt, der vom ersten Winkelbereich verschieden ist.

Durch geschickte Wahl des ersten Winkelbereichs und des zweiten Winkelbereichs kann die erfindungsgemäße Vorrichtung beispielsweise für beide oben beschriebenen Bewegungsabläufe verwendet werden. Bückt sich der Träger der orthopädietechnischen Einrichtung beispielsweise nur ein wenig oder arbeitet in gebeugter Haltung, entspricht dies vorzugsweise dem ersten Winkelbereich, sodass der erste passive Aktuator die benötigte Kraft aufbringt. Bückt sich der Träger der orthopädietechnischen Einrichtung jedoch beispielsweise um etwas vom Boden aufzuheben, entspricht der dabei entstehende Winkel zwischen dem Oberschenkelelement und dem Oberkörperelement vorzugsweise dem zweiten Winkelbereich, sodass der zweite passive Aktuator die Kraft aufbringt.

Vorzugsweise überlappen sich der erste Winkelbereich und der zweite Winkelbereich. Mit anderen Worten existieren folglich Winkel zwischen dem Oberschenkelelement und dem Oberkörperelement, in denen beide passiven Aktuatoren eine Kraft aufbringen.

Vorzugsweise weist der erste passive Aktuator und/oder der zweite passive Aktuator wenigstens einen mechanischen Energiespeicher und/oder einen Dämpfer auf. Dies kann beispielsweise ein elastisches Element, beispielsweise ein Federelement, bevorzugt eine Zugfeder sein. Der erste passive Aktuator und/oder der zweite passive Aktuator kann ausgebildet sein, um über den jeweiligen Winkelbereich, in dem der jeweilige Aktuator die Kraft aufbringt, eine konstante Kraft zu übertragen. Dazu kann der jeweilige Aktuator beispielsweise eine Konstantkraftfeder aufweisen. Alternativ oder zusätzlich dazu kann jedoch der Aktuator auch so ausgebildet sein, dass innerhalb des jeweiligen Winkelbereichs nicht eine konstante Kraft, sondern beispielsweise eine mit sinkendem Winkel steigende Kraft aufgebracht wird. Ein kleiner werdender Winkel bedeutet eine stärkere Beugung, sodass in diesem Fall die vom jeweiligen Aktuator aufgebrachte Kraft steigt, je tiefer sich der Benutzer der Vorrichtung drückt. In einer weiteren Ausgestaltung kann die Kraft auch bei einem Winkel innerhalb des jeweiligen Winkelbereichs ihr Maximum aufweisen und bei größerem Winkel und bei kleineren Winkel abfallen.

Vorzugsweise sind der erste passive Aktuator und der zweite passive Aktuator verschieden ausgebildet. Insbesondere können die elastischen Elemente der beiden Aktuatoren unterschiedliche Elastizitäten, insbesondere verschiedene Federkonstanten, und/oder unterschiedliche Dämpfungen aufweisen. Zudem können sie unterschiedliche Längen aufweisen, wobei die Länge des elastischen Elementes dabei im entspannten Zustand gemessen wird.

Vorzugsweise sind der erste passive Aktuator und/oder der zweite passive Aktuator an wenigstens einem Angriffspunkt am Oberschenkelelement und/oder am Oberkörperelement angeordnet, der jeweils verstellbar ist. Auf diese Weise lässt sich der jeweilige vorbestimmte Winkelbereich, innerhalb dessen der jeweilige Aktuator die Kraft aufbringt, einstellen. Zudem kann eine Vorspannung des jeweiligen passiven Aktuators erreicht werden, sodass auch die Größe der jeweils aufzubringenden Kraft einstellbar ist.

Um unterschiedliche Kräfte aufbringen zu können, ist es dabei von Vorteil, wenn der erste passive Aktuator und der zweite passive Aktuator an unterschiedlichen Angriffspunkten am Oberschenkelelement und/oder am Oberkörperelement angeordnet sind und/oder unterschiedliche Längen aufweisen. Auf diese Weise lässt sich insbesondere bei montierter Vorrichtung leicht erkennen, welcher Aktuator in welchem Winkelbereich seine Kraft aufbringt und/oder welcher Aktuator eine größere oder kleinere Kraft aufbringt. Selbstverständlich ist es auch möglich, die beiden Aktuatoren am gleichen Angriffspunkt angreifen zu lassen oder zwei Aktuatoren mit gleicher Länge zu verwenden. Dies ist beispielsweise dann möglich, wenn die beiden Aktuatoren unterschiedliche Federkonstanten und/oder Elastizitäten aufweisen.

In einer bevorzugten Ausgestaltung verfügt das Oberkörperelement über ein erstes Kraftübertragungselement und das Oberschenkelelement über ein zweites Kraftübertragungselement. Die beiden Kraftübertragungselemente sind in Eingriff und außer Eingriff bringbar. Der erste mechanische Energiespeicher und der zweite mechanische Energiespeicher sind aufladbar und entladbar, in dem das Oberschenkelelement relativ zu dem Oberkörperelement verschwenkt wird, sofern sich das erste Kraftübertragungselement in Eingriff mit dem zweiten Kraftübertragungselement befindet. Andernfalls können das Oberschenkelelement und das Oberkörperelement gegeneinander verschwenkt werden, ohne dass einer der beiden mechanischen Energiespeicher mit Energie aufgeladen werden wird. Durch diese Ausgestaltung ist es möglich, das Oberschenkelelement relativ zum Oberkörperelement zu verschwenken, ohne dass der jeweilige Energiespeicher mit Energie aufgeladen wird. In diesem Zustand wird dann auch keine Kraft vom Energiespeicher, also dem jeweiligen passiven Aktuator, aufgebracht. Dies ist für bestimmte Bewegungsabläufe von Vorteil. Steigt der Benutzer der Vorrichtung beispielsweise eine Treppe, muss er dazu die Oberschenkel und damit auch die an den Oberschenkeln angeordneten Oberschenkelelemente anheben. Mit anderen Worten muss er jeweils ein Oberschenkelelement relativ zum Oberkörperelement verschwenken. Während die beiden Kraftübertragungselemente in diesem Zustand in Eingriff miteinander, würde beim Anheben des Oberschenkelelementes der mechanische Energiespeicher aufgeladen und beim Strecken des Beines auf der nächsthöheren Stufe der Treppe wieder entladen. Soll die Vorrichtung jedoch beim Treppensteigen keine Unterstützung liefern, ist es sinnvoll, bei dieser Bewegung die beiden Kraftübertragungselemente außer Eingriff zugelassen.

In vielen Fällen soll die durch die orthopädietechnische Einrichtung gewährte Unterstützung nur beim Heben oder sich aus der Hocke Aufrichten vorhanden sein. Um dies zu gewährleisten muss sichergestellt werden, dass die beiden Kraftübertragungselemente nur in diesen Zuständen in Eingriff miteinander kommen. Dies lässt sich beispielsweise erreichen, indem ein Beckenelement vorhanden ist, und die beiden Kraftübertragungselemente in Eingriff miteinander gebracht werden, sobald ein Winkel zwischen dem Beckenelement oder einem Bauteil des Beckenelementes und dem Oberkörperelement einen vorbestimmten Grenzwinkel überschreitet. Vorzugsweise verfügt die Einrichtung daher über ein Beckenelement, wobei das Oberkörperelement relativ zu dem Beckenelement bewegbar angeordnet ist. Das erste Kraftübertragungselement und das zweite Kraftübertragungselement werden in diesem Fall in Eingriff und außer Eingriff gebracht, indem das Oberkörperelement relativ zu dem Beckenelement bewegt wird. Unterschreitet der Winkel zwischen dem Beckenelement und dem Oberkörperelement einen vorbestimmten Grenzwinkel, werden die beiden Kraftübertragungselemente in Eingriff miteinander gebracht. Überschreitet der Winkel danach den vorbestimmten Grenzwinkel, werden die Kraftübertragungselemente wieder außer Eingriff gebracht.

In bevorzugten Ausgestaltungen sind der erste passive Aktuator und der zweite passive Aktuator an jeweils einem Krafteinleitungspunkt an jeweils einem Krafteinleitungshebel angeordnet. Dieser ist vorzugsweise am Beckenelement oder am Oberkörperelement positioniert. Die beiden passiven Aktuatoren greifen in diesen Ausgestaltungen vorzugsweise am Oberschenkelelement an, sind also mit einem ihrer Enden am Oberschenkelelement und mit dem anderen am jeweiligen Krafteinleitungshebel angeordnet. Befinden sich die beiden Kraftübertragungselemente außer Eingriff, können die Krafteinleitungshebel relativ zum Beckenelement frei verschwenkt werden. Wird in diesem Zustand das Oberschenkelelement relativ zum Beckenelement und damit auch relativ zum Oberkörperelement verschwenkt, folgen die Krafteinleitungshebel dieser Verschwenkung, sodass die passiven Aktuatoren und die in ihnen vorzugsweise enthaltenen mechanischen Energiespeicher nicht mit Energie aufgeladen werden. Es entsteht somit keine Kraft und keine Unterstützung.

Greifen die beiden Kraftübertragungselemente jedoch ineinander ein, so sind die Krafteinleitungshebel drehfest am Beckenelement positioniert und können der Schwenkbewegung des Oberschenkelelementes nicht mehr folgen. Der Abstand zwischen dem Krafteinleitungspunkt am Krafteinleitungshebel einerseits und dem Angriffspunkt am Oberschenkelelement andererseits nimmt bei der Bewegung folglich zu, sodass der mechanische Energiespeicher mit mechanischer Energie aufgeladen wird und eine unterstützende Kraft ausübt.

Vorzugsweise ist eine Orientierung und/oder eine Position der beiden Krafteinleitungshebel zueinander und/oder wenigstens einer der beiden, vorzugsweise jedoch beide Krafteinleitungspunkt, verstellbar. Durch die Bewegung, beispielsweise Verschwenkung, der beiden Krafteinleitungshebel relativ zueinander lässt sich der Winkelbereich, in dem der jeweilige passive Aktuator seine Kraft aufbringt, einstellen. Durch eine Verschiebung des Krafteinleitungspunktes am Krafteinleitungshebel, beispielsweise in Richtung auf die Schwenkachse des Oberschenkelelementes relativ zum Beckenelement oder von dieser weg, lässt sich die Stärke der aufzubringenden Kraft einstellen. Durch andere Verstellung des Krafteinleitungspunktes am Krafteinleitungshebel, beispielsweise in Umfangsrichtung bezüglich der oben genannten Schwenkachse, lässt sich auch eine Vorspannung des jeweiligen passiven Aktuators erreichen.

Vorzugsweise ist eine Vorspannung des ersten Aktuators und/oder eine Vorspannung des zweiten Aktuators einstellbar.

Vorzugsweise verläuft die vom ersten Aktuator ausgeübte Kraft und/oder die vom zweiten Aktuator ausgeübte Kraft von dem Winkel abhängt, insbesondere kurvenförmig, besonders bevorzugt sinusförmig.

Bevorzugt weisen die vom ersten Aktuator ausgeübte Kraft und die vom zweiten Aktuator ausgeübte Kraft bei verschiedenen Winkeln ein Maximum auf.

Vorzugsweise ist die von dem jeweiligen Aktuator bei Winkeln kleiner als der jeweilige vorbestimmte Winkelbereich Null, oder im Wesentlichen Null. Der Winkel ist dabei umso kleiner, je stärker der Oberkörper relativ zum Oberschenkel gebeugt wird.

Vorzugsweise greift der erste passive Aktuator und der zweite passive Aktuator an jeweils einem Krafteinleitungshebel an. Die beiden Krafteinleitungshebel sind dabei vorzugsweise längenverstellbar ausgebildet, sodass sich die Größe des vom jeweiligen Aktuators aufgebrachten Drehmoments oder die Stärke der jeweiligen Kraft einstellen lässt. Zudem oder alternativ dazu sind die Krafteinleitungshebel relativ zueinander und/oder relativ zu einem Beckenelement vertretbar ausgebildet, sodass sich die Lage des vorbestimmten ersten Winkelbereiches und/oder die Lage des vorbestimmten zweiten Winkelbereiches einstellen lassen. Beim Beugen des Oberkörperelementes relativ zum Oberschenkelelement wird der jeweilige Aktuator, der beispielsweise ein Federelement sein kann, mit mechanischer Energie aufgeladen und kann so die Kraft aufbringen. Dabei wird der Abstand zwischen dem ersten Ende des jeweiligen Aktuators und dem zweiten Ende des Aktuators größer.

Vorzugsweise verfügt die Einrichtung über einen Anschlag, der beispielsweise an einem Beckenelement angeordnet sein kann, und an den der erste passive Aktuator und/oder der zweite passive Aktuator anschlägt, wenn der jeweilige Krafteinleitungshebel eine bestimmte Position insbesondere relativ zum Oberschenkelelement erreicht hat. Damit wird erreicht, dass der jeweilige Aktuator zwar noch immer gespannt und mit mechanischer Energie aufgeladen ist, diese jedoch vorzugsweise direkt auf die Rotationsachse zwischen dem Oberkörperelement und dem Oberschenkelelement wirkt, sofern der Anschlag auf dieser Rotationsachse angeordnet ist. Damit wird zwar eine Kraft ausgeübt, die jedoch nicht mehr zu einem Drehmoment und damit nicht mehr zu einer Unterstützung des Rückens führt.

Die orthopädietechnische Einrichtung zum Unterstützen eines unteren Rückens eines Benutzers, verfügt über wenigstens einen mechanischen Energiespeicher, ein Beckenelement, ein Oberkörperelement und ein Oberschenkelement, wobei der mechanische Energiespeicher aufladbar und entladbar ist, indem das Oberschenkelelement relativ zu dem Oberkörperelement verschwenkt wird. Vorzugsweise ist das Oberkörperelement mittels zweier Schienenelemente an dem Beckenelement angeordnet, wobei die Schienenelemente jeweils mit einem ersten Ende um wenigstens eine erste Schwenkachse schwenkbar an dem Beckenelement und mit einem dem ersten Ende entgegengesetzten zweiten Ende um wenigstens eine zweite Schwenkachse schwenkbar an dem Oberkörperelement angeordnet sind.

Das Beckenelement ist bevorzugt als Beckengurt oder Hüftgurt ausgebildet und verläuft folglich vollständig um den Rumpf in Höhe des Beckens oder der Hüfte herum. Zwischen den beiden ersten Enden der beiden Schienenelemente, die an dem Beckenelement angeordnet sind, erstreckt sich ein Teil des Beckenelement des, das vorzugsweise bei der Verwendung der Einrichtung nicht oder zumindest nahezu nicht längenveränderlich ausgebildet ist. **In** einer konstruktiv einfachen und daher bevorzugten Ausgestaltung ist auch der Abstand zwischen dem ersten Ende und dem zweiten Ende des jeweiligen Schienenelementes bei der Verwendung der Einrichtung nicht oder nahezu nicht längenveränderlich ausgebildet. Gleiches gilt für den Abstand zwischen den beiden zweiten Enden der Schienenelemente. Auf diese Weise ergibt sich ein Parallelogramm, das aufgrund der gelenkigen Anordnung der jeweiligen Teile beweglich ausgebildet ist. In einer bevorzugten Ausgestaltung wird erreicht, dass die Bewegungsfreiheit des Oberkörpers des Benutzers und insbesondere der Wirbelsäule des Benutzers nicht oder zumindest nahezu nicht eingeschränkt wird.

Vorzugsweise ist wenigstens eine der genannten Größen einstellbar ausgebildet. Die jeweilige Größe kann so auf eine Körpergröße des Benutzers angepasst werden. Sie wird nach dem Einstellen auf den individuell gewünschten Wert eingestellt und dann derart fixiert, beispielsweise arretiert, dass sie sich bei der Verwendung der Einrichtung nicht oder zumindest nahezu nicht ändert. Vorzugsweise sind mehrere, besonders bevorzugt alle genannten Größen auf diese Weise einstellbar und arretierbar.

Besonders bevorzugt ist das Oberkörperelement derart an dem Beckenelement angeordnet, dass die Lateralflexion der Wirbelsäule und die Rotation der Wirbelsäule um eine in der Sagittalebene liegende Rotationsachse möglich ist. In diesem Fall ist die Bewegungsfreiheit der Wirbelsäule des Benutzers in keiner Weise eingeschränkt, sodass alle Bewegungen, die der Benutzer der orthopädietechnischen Einrichtung mit seiner Wirbelsäule ohne die orthopädietechnische Einrichtung durchführen kann, auch mit der orthopädietechnischen Einrichtung durchführbar sind.

Unter einer in einer Sagittalebene liegenden Rotationsachse wird insbesondere eine vertikale Rotationsachse verstanden, die bei einem aufrecht stehenden Benutzer in der Medianebene, und damit in der mittleren Sagittalebene liegt. Sie könnte auch als Längsachse der Wirbelsäule bezeichnet werden, wobei die Wirbelsäule eines Menschen aufgrund ihrer geometrischen Ausgestaltung keine Längsachse im mathematischen Sinne aufweist. Selbstverständlich sind auch parallel zu dieser Achse verschobene Rotationsachsen in einer Sagittalebene liegend.

Wird die Bewegungsfreiheit der Wirbelsäule des Benutzers durch die orthopädietechnische Einrichtung nicht eingeschränkt, wird darunter insbesondere verstanden, dass eine Flexion und eine Extension möglich sind. Diese Bewegungen werden auch als Ventralflexion und Dorsalextension oder Inklination und Reklination bezeichnet. Eine Flexion ist das Vorneigen des Oberkörpers und damit der Wirbelsäule und des Kopfes, während die Extension die entgegengesetzte Bewegung ist. Zudem werden in diesem Fall auch andere Bewegungen des Oberkörpers und damit der Wirbelsäule, beispielsweise die Lateralflexion und die Rotation durch die orthopädietechnische Einrichtung nicht eingeschränkt.

Vorzugsweise werden auch Bewegungen der Wirbelsäule, insbesondere eine Neigung der Wirbelsäule zur Seite und/oder nach vorn und hinten und/oder eine Verdrehung der Wirkbelsäule um ihre Längsachse, nicht durch die orthopädietechnische Einrichtung behindert, eingeschränkt oder unmöglich gemacht. Vorzugsweise werden alle diese hier beschriebenen Bewegungen durch die orthopädietechnische Einrichtung weder in ihrem maximalen Bewegungsausschlag, noch in einer Bewegungsreihenfolge eingeschränkt.

Wird das Oberschenkelelement relativ zum Oberkörperelement in eine erste Richtung verschwenkt, wird der mechanische Energiespeicher, der beispielsweise ein elastisches Element, beispielsweise eine Zugfeder, sein kann, mit Energie aufgeladen. Diese erste Richtung entspricht beispielsweise dem Anheben des Oberschenkelelementes beispielsweise um eine Treppenstufe zu erklimmen. Bevorzugt ist die Einrichtung beim Treppensteigen jedoch in einem deaktiven Zustand, so dass beim Treppensteigen keine unterstützende Kraft aufgebracht wird. Auch durch das Vorbeugen (Flexion) des Oberkörpers wird das Oberkörperelement relativ zum Oberschenkelelement entsprechende verschwenkt. Die erste Richtung wird folglich dadurch gekennzeichnet, dass ein Winkel zwischen dem Oberschenkelelement und dem Oberkörperelement durch das Verschwenken abnimmt.

Die Energie, mit der der mechanische Energiespeicher aufgeladen wird, kann beispielsweise eine elastische oder potentielle Energie sein. In diesem Zustand übt der mechanische Energiespeicher vorzugsweise eine Kraft auf das Oberschenkelelement und/oder das Oberkörperelement aus, die in die der ersten Richtung entgegengesetzte zweite Richtung wirkt. Wird das Oberschenkelelement relativ zum Oberkörperelement in diese zweite Richtung verschwenkt, wird der mechanische Energiespeicher entladen und die so abgegebene Energie unterstützt die Bewegung des Oberschenkelelementes relativ zum Oberkörperelement. Bei dieser zweiten Richtung handelt es sich folglich beispielsweise um das Absenken des Oberschenkelelementes oder eine Streckung des Beines oder ein Aufrichten (Extension) des Oberkörpers. In all diesen Bewegungen wird das Oberschenkelelement relativ zum Oberkörperelement in die zweite Richtung verschwenkt.

Möchte der Benutzer der orthopädietechnischen Einrichtung beispielsweise einen schweren Gegenstand heben, geht er dafür in die Knie und greift den Gegenstand. Dabei werden beide Oberschenkel und damit auch das jeweilige Oberschenkelelement relativ zum Oberkörper und damit zum Oberkörperelement in die erste Richtung verschwenkt. Der Winkel zwischen dem Oberschenkel und dem Oberkörper nimmt ab. Dadurch wird der mechanische Energiespeicher potentielle Energie geladen. Zum Anheben des Gegenstandes muss der Benutzer der orthopädietechnischen Einrichtung nun die Beine strecken, wobei der Oberschenkel relativ zum Oberkörper in die entgegengesetzte zweite Richtung verschwenkt wird. Die im mechanischen Energiespeicher gespeicherte potentielle Energie wird dabei abgegeben und unterstützt auf diese Weise die entsprechende Bewegung.

Vorzugsweise verlaufen die ersten Schwenkachsen zumindest im Wesentlichen in Frontalebenen, vorzugsweise in einer gemeinsamen Frontalebene. Besonders bevorzugt verlaufen die ersten Schwenkachsen durch die Hüftgelenke des Benutzers, sodass die ersten Enden der Schienenelemente lateral, also außen, angeordnet sind. Die zweiten Enden der Schienenelemente hingegen sind dorsal, also hinten, am Oberkörperelement positioniert. Die Schienenelemente verlaufen bevorzugt derart, dass das erste Ende relativ zum zweiten Ende um 90° gedreht wird. Dabei sind die Schienenelemente vorzugsweise spiegelsymmetrisch zueinander ausgebildet und angeordnet.

Vorzugsweise verlaufen die zweiten Schwenkachsen zumindest im Wesentlichen in Sagittalebene. Dabei verlaufen sie insbesondere bevorzugt von dorsal nach ventral, also von hinten nach vorn. Auf diese Weise ist es möglich, auch ein Neigen des Körpers und der Wirbelsäule zur Seite zu ermöglichen, ohne die Bewegungsfreiheit einzuschränken.

In einer bevorzugten Ausgestaltung verfügen die Schienenelemente über wenigstens zwei Teilschienen, die aneinander um eine dritte Schwenkachse schwenkbar angeordnet sind. Die dritten Schwenkachsen verlaufen dabei vorzugsweise im Wesentlichen in Sagittalebenen. Besonders bevorzugt verlaufen Sie im angelegten Zustand der orthopädietechnischen Einrichtung im Wesentlichen parallel zu den zweiten Schwenkachsen, wenn der Benutzer der orthopädietechnischen Einrichtung aufrecht steht. Die Schwenkgelenke, die eine Bewegung der Teilschienen relativ zueinander ermöglichen, sind bevorzugt näher am ersten Ende als am zweiten Ende der jeweiligen Schienenelemente angeordnet. Besonders bevorzugt befinden sich diese Gelenke seitlich des Körpers des Benutzers, sodass eine gedachte Verlängerung der dritten Schenkachsen am Körper des Benutzers vorbei führt.

Vorzugsweise sind die zweiten Enden der Schienenelemente im angelegten Zustand der orthopädietechnischen Einrichtung im Bereich der Schulterblätter, besonders bevorzugt im Bereich der unteren Winkel der Schulterblätter des Benutzers angeordnet. In diesem Bereich findet bei einer Beugung der Wirbelsäule nach rechts oder links die stärkste Abweichung von einer geraden Linie statt, so dass die Gelenke, die in diesem Bereich die zweiten Enden mit dem Oberkörperelement verbinden, optimal positioniert sind.

In einer bevorzugten Ausgestaltung ist ein Abstand zwischen Gelenken, mit denen die zweiten Enden der Schienenelemente am Oberkörperelement angeordnet sind, einstellbar. Die Gelenke sind bevorzugt verlagerbar an dem Oberkörperelement angeordnet. Dies wird beispielsweise erreicht, indem das jeweilige Gelenk an einem Schieber angeordnet ist, der entlang einer Führung, beispielsweise einem Langloch oder einer Kulisse, die in oder an dem Oberkörperelement angeordnet ist, verschiebbar ist.

Bevorzugt sind die zweiten Enden der Schienenelemente derart an dem Oberkörperelement angeordnet, dass sie um zwei verschiedene Schwenkachsen schwenkbar sind, von denen eine vorzugsweise in dorsal-ventral-Richtung und eine in medial-lateral-Richtung verläuft. Die erste dieser beiden Schwenkachsen erlaubt es dem Benutzer der orthopädietechnischen Einrichtung, seinen Oberkörper nach rechts und links zu neigen, während die zweite der beiden Schwenkachsen benötigt wird, um den Oberkörper des Benutzers nach vorn oder hinten zu beugen.

In bevorzugten Ausführungsformen sind die zweiten Enden der Schienenelemente mittels Kugelgelenken an dem Oberkörperelement angeordnet. Dadurch die die Bewegungsfreiheit weiter gesteigert und die Akzeptanz der orthopädietechnischen Einrichtung beim Benutzer weiter erhöht.

Das Oberkörperelement verläuft im angelegten Zustand der orthopädietechnischen Einrichtung vorzugsweise vollständig um den Oberkörper des Benutzers herum. Es ist dabei bevorzugt so formstabil ausgebildet, dass sich sein Durchmesser in medial-lateral-Richtung beim Beugen des Oberkörpers nicht oder im Wesentlichen nicht ändert. Soll der wenigstens eine mechanische Energiespeicher mit Energie aufgeladen werden, muss das Oberkörperelement relativ zu dem Oberschenkelelement verschwenkt werden. Gegebenenfalls muss noch eine Aktivierungsvorrichtung betätigt werden, was beispielsweise durch eine Bewegung des Oberkörperelementes relativ zu dem Beckenelement geschehen kann. Wird der Energiespeicher, der beispielsweise ein Federelement aufweist, aufgeladen, muss dazu eine Kraft ausgeübt werden, die durch das Beugen des Oberkörpers hervorgerufen werden kann. Der Oberkörper übt dann in den genannten Ausführungsbeispielen eine Zugkraft auf das Oberkörperelement aus.

Vorzugsweise weist das Oberkörperelement einen Brustabschnitt auf, der im angelegten Zustand der orthopädietechnischen Einrichtung an wenigstens zwei voneinander beabstandeten Stellen auf unterschiedlichen Seiten eines Brustbeines des Benutzers an der Brust des Benutzers anliegt. Über diese Stellen wird die Kraft von dem Oberkörper auf das Oberkörperelement übertragen. Dies ist natürlich auch möglich, wenn das Oberkörperelement nur an einer einzigen Stelle oder an mehr als zwei Stellen mit der Brust des Benutzers in Kontakt kommt.

Zum Aufladen des Energiespeichers mit Energie wird folglich eine Zugkraft auf das Oberkörperelement durch den Oberkörper und damit durch den Benutzer der orthopädietechnischen Einrichtung ausgeübt. Wird der Energiespeicher entladen, wird eine Zugkraft durch das Oberkörperelement auf den Oberkörper ausgeübt, durch die der untere Rücken des Benutzers beispielsweise beim Aufrichten, unterstützt wird. Die Zugkraft wird dabei vorzugsweise über die Schienenelemente auf das Oberkörperelement und von diesen auf den Oberkörper übertragen. Da die Schienenelemente dorsal, also am Rücken, des Benutzers angeordnet sind, wird die Zugkraft auf den dorsalen Anteil des Oberkörperelementes übertragen und von dort auf den frontalen Anteil des Oberkörperelementes gebracht. Über die Stellen, an denen dieser frontale Anteil mit dem Oberkörper in Kontakt kommt, vorzugsweise also rechts und links des Brustbeines des Benutzers, wird diese Zugkraft auf den Oberkörper übertragen. Dabei stellt die ausreichende Formstabilität sicher, dass es nicht zu einer Einschnürung des Oberkörpers des Benutzers kommt, wenn auf den dorsalen Anteil des Oberkörperelementes die Zugkraft ausgeübt wird. Ist die Formstabilität zu klein, wird die eigentlich von frontal nach dorsal durch die seitlich am Oberkörper vorbeiführenden Teile des Oberkörperelementes wie bei einer Schlinge, auf die eine Zugkraft ausgeübt wird, auf den Oberkörper übertragen. Dabei wird ein Teil der Kraft in eine medial wirkende Kraft übertragen, die zu schmerzhaften Effekten führen kann.

Vorzugsweise ist der Teil des Oberkörperelementes, der den Oberkörper umgibt, nicht vollständig formstabil, sondern weist eine geringe Flexibilität und vorzugsweise Elastizität auf. Dadurch ist sichergestellt, dass die orthopädietechnische Einrichtung und das Oberkörperelement für unterschiedliche Personen mit unterschiedlichen Brustumfängen geeignet ist und in der Größe einstellbar ausgebildet sein kann. Dabei ist es gegebenenfalls ausreichend, einzelne starre und unflexible Elemente auf flexible und vorzugsweise elastische Weise miteinander zu verbinden, also beispielsweise Halbschalen oder Schalenelemente zu verwenden, die Teile des Oberkörpers formstabil und starre umgeben. Alternativ dazu kann das Oberkörperelement auch vollständig ohne starre Elemente ausgebildet sein.

In einer bevorzugten Ausgestaltung verfügt die orthopädietechnische Einrichtung über ein erstes und ein zweites Oberschenkelelement und einen ersten und einen zweiten mechanischen Energiespeicher. Dabei ist der erste mechanische Energiespeicher aufladbar und entladbar, indem das erste Oberschenkelelement relativ zu dem Oberkörperelement verschwenkt wird. Der zweite mechanische Energiespeicher ist aufladbar und entladbar, indem das zweite Oberschenkelelement relativ zu dem Oberkörperelement verschwenkt wird. Durch diese Ausgestaltung ist eine Bewegung der Oberschenkel relativ zum Oberkörperelement unabhängig voneinander möglich. Nur auf den Oberschenkel, der relativ zum Oberkörperelement verschwenkt wurde, wird durch den mechanischen Energiespeicher eine Kraft ausgeübt.

Vorteilhafterweise ist jedes Oberschenkelelement mittels einer Gelenkanordnung um eine Gelenkachse schwenkbar an dem Beckenelement angeordnet. Vorzugsweise wird dabei die Gelenkanordnung so positioniert, dass die Gelenkachse durch ein Hüftgelenk des Benutzers verläuft.

Bevorzugt verfügt das Oberschenkelelement über wenigstens ein Anlageelement zum Anlegen an den Oberschenkel und über wenigstens ein Druckkraftübertragungselement, durch das das Anlageelement mit der Gelenkanordnung verbunden ist. In einer bevorzugten Ausführungsform ist das Druckkraftübertragungselement eine Stange oder eine Schiene und ist besonders bevorzugt ergonomisch geformt. Vorzugsweise ist das Anlageelement durch jeweils längstens ein Druckkraftübertragungselement mit jeder Gelenkanordnung verbunden.

Vorteilhafterweise ist jedes der verwendeten Schienenelemente um wenigstens zwei Schwenkachsen schwenkbar an dem Oberkörperelement angeordnet, wobei wenigstens zwei der Schwenkachsen vorzugsweise senkrecht auf einander stehen.

Besonders bevorzugt ist wenigstens eines der Schienenelemente mittels eines Kugelgelenkes an dem Oberkörperelement angeordnet. Vorzugsweise sind alle Schienenelemente mittels jeweils eines Kugelgelenkes an dem Oberkörperelement angeordnet.

Bevorzugt ist wenigstens ein Schienenelement, besonders bevorzugt jedoch jedes Schienenelement, um eine Bewegungsachse schwenkbar an der jeweiligen Gelenkanordnung, die an dem Beckenelement angeordnet ist, angeordnet, wobei Bewegungsachse vorzugsweise senkrecht auf der Gelenkachse der jeweiligen Gelenkanordnung steht.

Die verschiedenen bewegbaren Ausgestaltungen wird erreicht, dass den Bewegungen des Oberkörpers und insbesondere der Wirbelsäule des Benutzers gefolgt werden kann, und unabhängig von der Position des Oberkörperelementes relativ zum Beckenelement und/oder relativ zu dem wenigstens einen Oberschenkelelement die durch den mechanischen Energiespeicher im geladenen Zustand aufgebrachte Kraft wirken kann.

In einer besonders bevorzugten Ausgestaltung ist das wenigstens eine Schienenelement längenveränderbar ausgebildet. Besonders bevorzugt sind alle Schienenelemente längenveränderbar. Dadurch kann die orthopädietechnische Einrichtung für unterschiedlich große Personen verwendet werden. Bevorzugt ist das längenveränderbare Schienenelement in unterschiedlichen Längeneinstellungen fixierbar, sodass die Länge zwar einstellbar, danach jedoch unveränderbar ist.

Vorteilhafterweise weist der mechanische Energiespeicher wenigstens ein Federelement, einen Druckspeicher, ein pneumatisches und/oder hydraulisches System und/oder einen hydraulischen Energiespeicher auf. Auch elastische Elemente in Form von elastischen Seilen, beispielsweise Gummiseilen, sind vorstellbar. Selbstverständlich sind auch andere Elemente, beispielsweise Gasdruckfedern oder Druckfedern, denkbar, für die eine Umlenkung verwendet wird, um aus der durch die Druckfeder bereitgestellten Druckkraft eine Zugkraft zu machen.

Der mechanische Energiespeicher kann an unterschiedlichsten Positionen der Vorrichtung angeordnet sein. Vorteilhafterweise wird eine Position gewählt, an der der für den Energiespeicher benötigte Bauraum vorhanden ist und der Energiespeicher selbst bei Bewegungen des Beins des Benutzers nicht stört. So kann er beispielsweise am Oberschenkel angeordnet sein.

Zum Anordnen des Oberkörperelementes an dem Oberkörper des Benutzers eignet sich besonders ein Schulterelement zum Anlegen an die Schulter, das beispielsweise in Form von Rucksackträgern oder Hosenträgern ausgebildet sein kann. Es erlaubt eine besonders kleine Bauform der orthopädietechnischen Einrichtung.

Das Oberschenkelelement verfügt vorzugsweise über eine Oberschenkelschale, die vorzugsweise an einem Abstandselement angeordnet ist. Dieses Abstandselement ist als Teil des Oberschenkelelementes vorteilhafterweise mit dem Beckenelement verbunden. Die Längen des beispielsweise als Schiene oder Stab ausgebildeten Druckkraftübertragungselementes und des gegebenenfalls auch als Stab oder Schiene ausgebildeten Abstandselementes sind dabei vorzugsweise so gewählt, dass der gesamte Winkelbereich der möglichen Bewegung des Oberschenkels des Trägers abgedeckt wird. Die Oberschenkelschale ist vorzugsweise gelenkig an dem Abstandselement angeordnet, um einen größtmöglichen Tragekomfort zu erreichen.

In einer bevorzugten Ausgestaltung ist der passive Aktuator eingerichtet, die Kraft in Abhängigkeit von einer Position und/oder einer Orientierung des wenigstens einen Beinstützelementes relativ zum Beckenelement und/oder zum Oberkörperelement aufzubringen.

In einer bevorzugten Ausgestaltung ist an dem Oberschenkelelement, bevorzugt an jedem Oberschenkelelement die Oberschenkelschale zum Anlegen an den Oberschenkel des Benutzers angeordnet. Diese ist vorzugsweise gepolstert ausgebildet, um ein möglichst angenehmes Tragegefühl zu vermitteln. Die Oberschenkelschale ist vorzugsweise mittels eines Kugelgelenkes angeordnet. Dadurch wird eine möglichst umfassende Bewegungsfreiheit gegenüber dem Rest der Einrichtung erreicht, die insbesondere bei Bewegungen des Benutzers von Vorteil ist. Mittels des Kugelgelenkes kann die Oberschenkelschale direkt an einem Schienenelement oder Abstandselement des Oberschenkelelements angeordnet sein. Alternativ dazu ist sie an einem Haltebügel positioniert.

Vorzugsweise ist die Oberschenkelschale relativ zu dem Oberschenkelelement um eine Rotationsachse schwenkbar, bevorzugt gegen eine Kraft eines Federelementes schwenkbar, wobei sich die vorzugsweise Rotationsachse in medial-lateral-Richtung erstreckt. Dies wird besonders einfach durch die Positionierung der Oberschenkelschale an dem Haltebügel erreicht, der um die Rotationsachse schwenkbar an einem weiteren Bauteil des Oberschenkelelementes angeordnet ist.

Mithilfe der beigefügten Zeichnungen werden nachfolgend einige Ausführungsbeispiele der vorliegenden Erfindung näher erläutert. Es zeigen
- Figuren 1, 3 und 4: - schematische Darstellungen eines Teils einer Sicherungseinrichtung gemäß einem ersten Ausführungsbeispiel der vorliegenden Erfindung
- Figuren 2 und 5 bis 8: - verschiedene Formen von Zähnen und
- Figuren 9 bis 11: - weitere Ausführungsformen von Sicherungseinrichtungen.

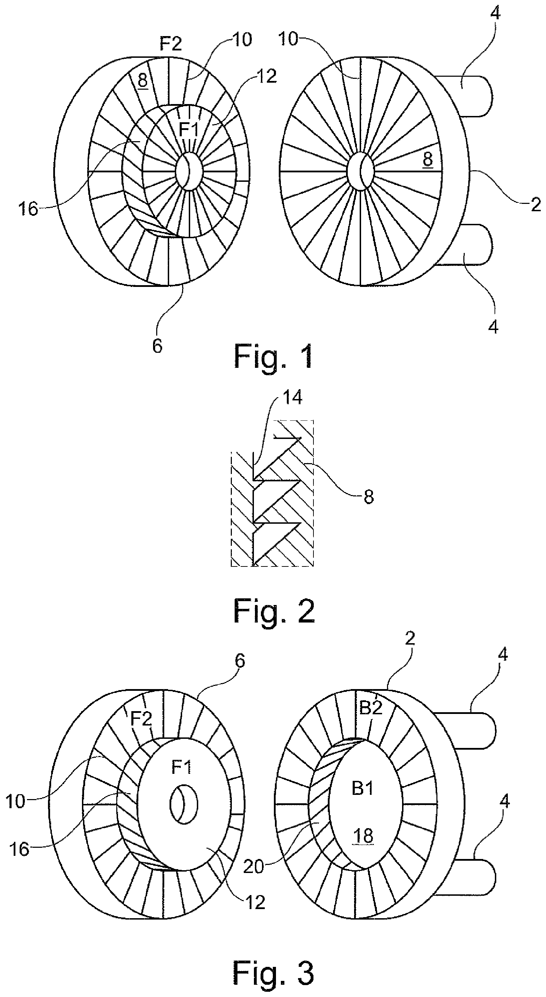

Figur 1 zeigt schematisch verschiedene Elemente einer orthopädietechnischen Einrichtung gemäß einem Ausführungsbeispiel der vorliegenden Erfindung. Gezeigt ist ein erstes Formschlusselemente, das als erstes Zahnrad 2 ausgebildet ist, das entlang von Führungsstäben 4 an dem Bauteil, an dem es angeordnet ist, verschiebbar ist. Es wird entlang dieser Führungsstäben 4 verschoben, wenn das erste Zahnrad 2 mit dem zweiten Formschlusselement, das als zweites Zahnrad 6 in Eingriff gebracht werden soll. Das erste Zahnrad 2 und das zweite Zahnrad 6 verfügen an ihren Stirnseiten 8 über schematisch angedeutete Zähne 10, die zueinander korrespondierend ausgebildet sind. Aus der Stirnseite 8 des zweiten Zahnrades 6 ragt eine Führungsspindel 12 in axialer Richtung hervor, an deren Stirnseite 14 ebenfalls Zähne angeordnet sind. Die Zahnung der Stirnseite 14 der Führungsspindel 12 entspricht der Zahnung der Stirnseite 8 des zweiten Zahnrades 6. An einer Mantelfläche 16 der Führungsspindel 12 ist ebenfalls eine Verzahnung angeordnet, die dafür sorgt, dass die Führungsspindel 12 in Rotation versetzt wird, wenn sie in axialer Richtung bezüglich des zweiten Zahnrades 6 verschoben wird, bis sie in dem zweiten Zahnrad 6 aufgenommen ist.

Wird das erste Zahnrad 2 entlang der Führungsstäben 4 auf das zweite Zahnrad 6 zubewegt, greifen zunächst die Zähne der Stirnseite 14 der Führungsspindel 12 in die Zähne 10 der Stirnseite 8 des ersten Zahnrades 2. Die Führungsspindel 12 wird dann in das zweite Zahnrad 6 geschoben und dabei aufgrund der Zähne der Mantelfläche 16 in Rotation versetzt. Das erste Zahnrad 2 ist so gelagert, dass es der nur geringen Rotation der Führungsspindel 12 folgen kann, sodass es die optimale Position bezüglich des zweiten Zahnrades 6 erreicht, sobald die Führungsspindel 12 in dem zweiten Zahnrad 6 aufgenommen wurde.

Figur 2 zeigt schematisch eine Ausführungsform der unterschiedlichen Zähne. Links ist die Stirnfläche 14 der Führungsspindel 12 und rechts die Stirnfläche 8 des ersten Zahnrades 2 gezeigt. Durch die sehr unterschiedlich ausgebildeten Zähne wird sichergestellt, dass unabhängig von der Position, in der das erste Zahnrad 2 auf die Führungsspindel 12 trifft, die jeweiligen Zähne immer in Eingriff miteinander kommen.

Figur 3 zeigt eine andere Ausgestaltung. Das erste Zahnrad 2 verfügt über eine zentrale Bohrung 18, deren Innenwandung 20 mit Nuten oder einer Zahnung, die auch als Innengewinde ausgebildet sein kann, versehen ist. Auch die in Figur 1 gezeigte Zahnung an der Mantelfläche 16 kann in Form eines Außengewindes vorhanden sein. Im in Figur 3 gezeigten Ausführungsbeispiel verfügt das zweite Zahnrad 6 über eine Führungsspindel 12, die jedoch nicht relativ zum zweiten Zahnrad 6 verschiebbar angeordnet ist. Vielmehr ist die Mantelfläche 16 der Führungsspindel 12 mit einem Außengewinde ausgebildet, das zum Innengewinde der Innenwandung 20 der zentralen Bohrung 18 korrespondierend ausgestaltet ist. Wird in dieser Ausgestaltung das erste Zahnrad 2 entlang der Führungsstäbe 4 auf das zweite Zahnrad zu verschoben, greift das Außengewinde der Mantelfläche 16 in das Innengewinde der Innenwandung 20 der zentralen Bohrung 18 des ersten Zahnrades 2. Durch die weitere Verschiebung des ersten Zahnrades 2 in Richtung auf das zweite Zahnrad 6 kommt es dabei zu einer Drehung des ersten Zahnrades 2 relativ zum zweiten Zahnrad 6, wodurch wieder erreicht wird, dass die Zähne 10 des ersten Zahnrades 2 möglichst optimal in die Zähne 10 des zweiten Zahnrades 6 eingreifen.

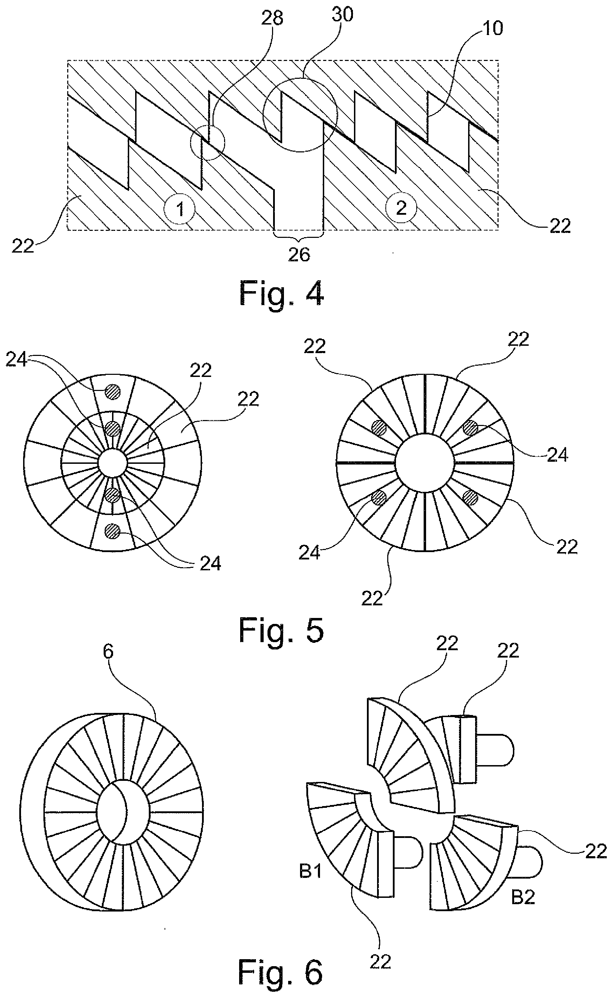

Die Figuren 4 und 5 zeigen eine schematische Darstellung von Teilen einer Sicherungseinrichtung, bei der eines der Zahnräder Teilzahnräder aufweist. Im linken Bereich der Figur 5 verfügt eines der Zahnräder über zwei Teilzahnräder 22, die koaxial angeordnet sind. Über schematisch angeordnete elastische Elemente 24 sind die Teilzahnräder 22 in axialer Richtung, also senkrecht zur Zeichenebene, verschieblich angeordnet. Die Zähne der Teilzahnräder 22 sind dabei leicht versetzt zueinander angeordnet. Der Versatz beträgt insbesondere vorzugsweise eine halbe Zahnlänge. Greift nun ein Zahnrad in die Zähne dieser Teilzahnräder 22, die bevorzugt in axialer Richtung unterschiedlich weit hervorstehenden, ergibt sich die in Figur 4 gezeigte Situation. Die Teilzahnräder 22 verfügen jeweils über Zähne, die durch den Versatz 26 voneinander getrennt sind und deswegen unterschiedlich weit in die Zähne 10 des jeweils anderen Zahnrades eingreifen. Im Bereich 28 ist nur ein sehr geringer Kontakt zwischen den Zähnen des Teilzahnrades 22 und den Zähnen 10 des jeweiligen Zahnrades vorhanden. Wird die zu übertragende Kraft zu, rutschen die Zähne hier voneinander ab. Die beiden Bauteile können jedoch nur soweit gegeneinander verschoben werden, bis im Bereich 30 die Zähne des anderen Teilzahnrades 22 mit den Zähnen 10 des Zahnrades in Eingriff sind. Da die Teilzahnräder 22 in axialer Richtung gegeneinander verschoben sind, greifen die Zähne der verschiedenen Teilzahnräder unterschiedlich weit in die Zähne des Zahnrades ein.

Im rechten Teil der Figur 4 verfügt das Zahnrad über vier Teilzahnräder 22, die jeweils über ein elastisches Element 24 in axialer Richtung elastisch gelagert sind. Figur 6 zeigt schematisch eine Kombination eines aus vier Teilzahnräder 22 bestehenden ersten Zahnrades 2 und eines zweiten Zahnrades 6, dass ein einfaches Stirnzahnrad ist. Die vier Teilzahnräder 22 entsprechen der in Figur 5 gezeigten Anordnung und sind in axialer Richtung stark versetzt und nicht maßstabsgetreu dargestellt angeordnet.

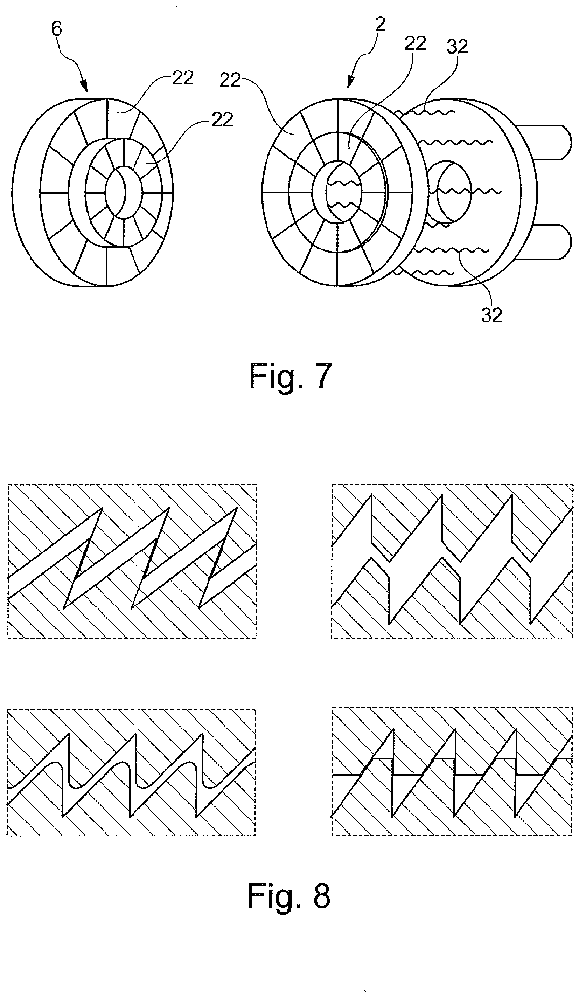

Figur 7 zeigt eine weitere Ausgestaltung. Das zweite Zahnrad 6 weist zwei Teilzahnräder 22 auf, die axial zueinander leicht versetzt angeordnet, zueinander jedoch fixiert sind. Das erste Zahnrad 2 verfügt ebenfalls über zwei Teilzahnräder 22 die wie die Teilzahnräder 22 des zweiten Zahnrades 6 koaxial zueinander angeordnet sind und über angedeutete Federelemente 32 axial verschieblich sind. Werden die gezeigten Zahnräder 2, 6 aufeinander zu bewegt, greift in der gezeigten Ausführungsform zunächst das innere Teilzahnrad 22 in die Zähne des inneren Teilzahnrades 22. Erst beim weiteren Verschieben des ersten Zahnrades 2 in Richtung auf das zweite Zahnrad 6 greifen auch die äußeren Teilzahnräder 22 ineinander ein. Aufgrund der versetzten Zähnung entsteht die in Figur 4 schematisch dargestellte Situation. Entspricht der Eingriff der inneren Teilzahnräder 22 ineinander der im Bereich 28 gezeigten Situation, ist sichergestellt, dass die äußeren Teilzahnräder 22 entsprechen der im Bereich 30 der Figur 4 gezeigten Situation ineinander eingreifen.

Figur 8 zeigt unterschiedliche Zahnformen, die verwendet werden können.

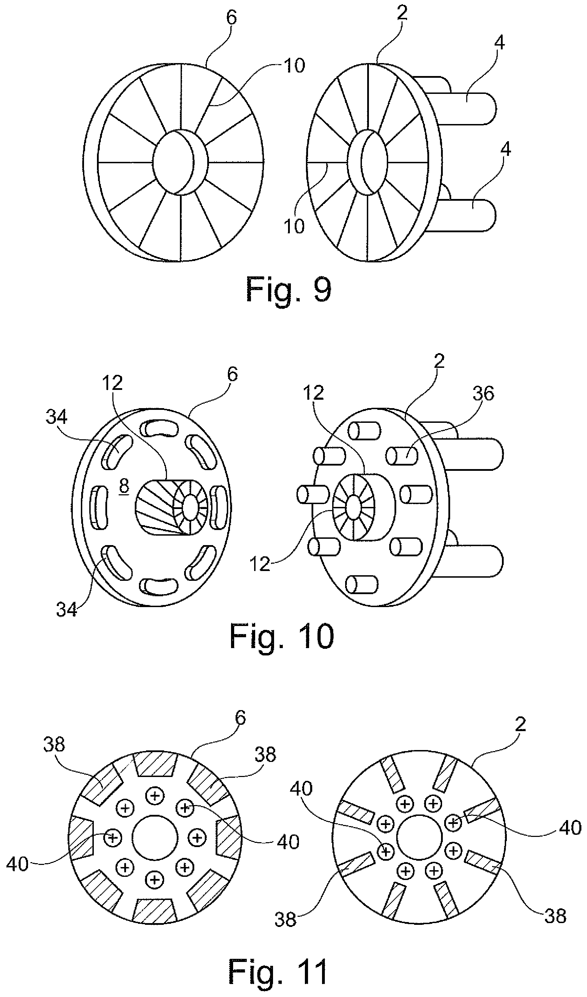

Figur 9 zeigt das erste Zahnrad 2 und das zweite Zahnrad 6 wie in Figur 1 dargestellt. Das erste Zahnrad 2 ist wieder entlang der Führungsstäbe 4 verschieblich angeordnet. Die Zähne 10 im gezeigten Ausführungsbeispiel sind als Hinterschnittzähnung ausgebildet.

In Figur 10 verfügen sowohl das erste Zahnrad 2 als auch das zweite Zahnrad 6 über jeweils eine Führungsspindel 12 die aus dem jeweiligen Zahnrad 2, 6 stirnseitig hervorragend. Die beiden Führungsspindel 12 verfügen über stirnseitige Vorsprünge und/oder Vertiefungen, die miteinander in Eingriff gebracht werden können. Anstelle der in den anderen Figuren gezeigten Verzahnung sind in der Stirnseite 8 des zweiten Zahnrades 6 Langlöcher 34 vorhanden, die so ausgebildet sind, dass Stifte 36, die aus der Stirnfläche des ersten Zahnrades 2 hervorstehenden, darin eingreifen können.

Figur 11 zeigt die Stirnseiten des ersten Zahnrades 2 des zweiten Zahnrades 4, die jeweils über Vorsprünge 38 verfügen zwischen denen sich Vertiefungen befinden, sodass die Vorsprünge 38 der beiden Zahnräder ineinander eingreifen können. Im mittleren Bereich der Stirnseiten sind Magnete 40 schematisch dargestellt, die so angeordnet sind, dass gleichnamige Pole aufeinander zu gerichtet sind. Es entsteht so eine abstoßende Wirkung, die minimal ist, wenn die vorzugsweise äquidistant angeordneten Magnete 40 des einen Zahnrades genau zwischen den Magneten des anderen Zahnrades angeordnet werden. Auch auf diese Weise kann eine Ausrichtung der beiden Zahnräder 2, 6 zueinander erfolgen.

### Bezugszeichenliste

- 2: erstes Zahnrad
- 4: Führungsstab
- 6: zweite Zahnrad
- 8: Stirnseite
- 10: Zahn
- 12: Führungsspindel
- 14: Stirnseite
- 16: Mantelfläche
- 18: zentrale Bohrung
- 20: Innenwandung
- 22: Teilzahnrad
- 24: elastisches Element
- 26: Versatz
- 28: Bereich
- 30: Bereich
- 32: Federelement
- 34: Langloch
- 36: Stift
- 38: Vorsprung
- 40: Magnet

## Patentansprüche

1. Orthopädietechnische Einrichtung, die
ein Gelenk mit
- einem ersten Gelenkelement, das
∘ einen ersten Gelenkarm mit einem ersten Formschlusselement (2) aufweist, und
- einem relativ zu dem ersten Gelenkelement verschwenkbaren zweiten Gelenkelement, das
∘ einen zweiten Gelenkarm und
∘ einen Kraftangriffshebel mit einem zweiten Formschlusselement (6) und
∘ einen mechanischen Energiespeicher aufweist, der zwischen dem Kraftangriffshebel und dem zweiten Gelenkarm angeordnet ist,
wobei der mechanische Energiespeicher aufladbar und entladbar ist, indem der erste Gelenkarm relativ zu dem zweiten Gelenkarm verschwenkt wird, wenn das erste Formschlusselement (2) mit dem zweiten Formschlusselement (6) in Eingriff ist,
**dadurch gekennzeichnet, dass**
die Einrichtung eine Sicherungseinrichtung aufweist, durch die sichergestellt ist, dass unabhängig von der Position des ersten Formschlusselementes (2) und des zweiten Formschlusselementes (6) relativ zueinander die beiden Formschlusselemente (2,6) derart in Eingriff bringbar sind, dass eine von dem aufgeladenen mechanischen Energiespeicher wirkende Kraft von dem zweiten Formschlusselement (6) auf das erste Formschlusselement (2) übertragen wird.

2. Orthopädietechnische Einrichtung nach Anspruch 1, **dadurch gekennzeichnet, dass** die Sicherungseinrichtung eingerichtet ist, beim in Eingriff Bringen oder nach dem in Eingriff Bringen der beiden Formschlusselemente (2,6) die Formschlusselemente (2,6) relativ zueinander zu drehen.

3. Orthopädietechnische Einrichtung nach Anspruch 2, **dadurch gekennzeichnet, dass** das erste Formschlusselement (2) und das zweite Formschlusselement (6) stirnseitige Vorsprünge und/oder Vertiefungen aufweisen und die Sicherungseinrichtung eine aus einem der Formschlusselemente (2, 6) axial hervorstehende Führungsspindel (12) aufweist, die Vorsprünge und/oder Vertiefungen aufweist, die eingerichtet ist, mit dem jeweils anderen Formschlusselement (6, 2) zusammenzuwirken.

4. Orthopädietechnische Einrichtung nach Anspruch 3, **dadurch gekennzeichnet, dass** die Führungsspindel (12) in axialer Richtung relativ zu dem Formschlusselement (2, 6), aus dem sie axial hervorsteht, verschiebbar ist, wobei die Führungsspindel (12) derart ausgebildet ist, dass beim axialen Verschieben die Führungsspindel (12) um ihre Längsachse gedreht wird, so dass ein Drehmoment auf das Formschlusselement (2, 6) ausgeübt wird, das mit den Vorsprüngen und/oder Vertiefungen der Führungsspindel (12) zusammenwirkt.

5. Orthopädietechnische Einrichtung nach einem der vorstehenden Ansprüche, **dadurch gekennzeichnet, dass** das erste Formschlusselement (2) und/oder das zweite Formschlusselement (6) wenigstens zwei Teilformschlusselemente (22) aufweist, die in axialer Richtung unabhängig voneinander bewegbar sind.

6. Orthopädietechnische Einrichtung nach Anspruch 5, **dadurch gekennzeichnet, dass** die wenigstens zwei Teilformschlusselemente (22) die gleichen Vorsprünge und/oder Vertiefungen aufweisen, die in Umfangsrichtung gegeneinander versetzt sind.

7. Orthopädietechnische Einrichtung nach Anspruch 5 oder 6, **dadurch gekennzeichnet, dass** die wenigstens zwei Teilformschlusselemente (22) in axialer Richtung voneinander beabstandet sind, wenn das erste Formschlusselement (2) und das zweite Formschlusselement (6) nicht miteinander in Eingriff sind.

8. Orthopädietechnische Einrichtung nach einem der vorstehenden Ansprüche, **dadurch gekennzeichnet, dass** das erste Gelenkelement ein Oberkörperelement und das zweite Gelenkelement ein Oberschenkelelement ist und die Einrichtung ein Beckenelement aufweist, wobei die beiden Formschlusselemente (2, 6) in Eingriff und außer Eingriff bringbar sind, indem das Oberkörperelement relativ zu dem Beckenelement bewegt wird.

9. Orthopädietechnische Einrichtung nach einem der Ansprüche 2 bis 8, **dadurch gekennzeichnet, dass** zumindest einige, bevorzugt alle Vorsprünge der beiden Formschlusselemente (2, 6) eine Hinterschnittverzahnung aufweisen.

10. Orthopädietechnische Einrichtung nach einem der vorstehenden Ansprüche, **dadurch gekennzeichnet, dass** die Formschlusselemente (2, 6) miteinander in Eingriff bringbar sind, indem eines der Formschlusselemente (2, 6) auf das andere Formschlusselement (2, 6) zu bewegt wird, das relativ zu dem Bauteil, an dem es angeordnet ist, in einer Richtung drehbar gelagert ist.

## Claims

1. An orthopaedic device comprising
a joint with
- a first joint element comprising
∘ a first joint arm with a first form-fitting element (2) and
- a second joint element that can be swivelled relative to the first joint element comprising
∘ a second joint arm and
∘ a force application lever with a second form-fitting element (6) and
∘ a mechanical energy store arranged between the force application lever and the second joint arm,
wherein the mechanical energy store can be charged and discharged by swivelling the first joint arm relative to the second joint arm when the first form-fitting element (2) is engaged with the second form-fitting element (6),
**characterised in that**
the device comprises a securing device which ensures that, irrespective of the position of the first form-fitting element (2) and the second form-fitting element (6) relative to each other, the two form-fitting elements (2,6) can be engaged in such a way that an acting force of the charged mechanical energy store is transmitted from the second form-fitting element (6) to the first form-fitting element (2).

2. The orthopaedic device according to claim 1, **characterised in that** the securing device is configured to rotate the form-fitting elements (2,6) relative to each other when the two form-fitting elements (2,6) are being engaged with one another or thereafter.

3. The orthopaedic device according to claim 2, **characterised in that** the first form-fitting element (2) and the second form-fitting element (6) comprise projections and/or recesses on the end face and that the securing device comprises a guide spindle (12) that protrudes axially from one of the form-fitting elements (2,6), said spindle comprising projections and/or recesses and that is configured to interact with the respective other form-fitting element (6,2).

4. The orthopaedic device according to claim 3, **characterised in that** the guide spindle (12) can be displaced in the axial direction relative to the form-fitting element (2,6) from which it axially protrudes, wherein the guide spindle (12) is designed in such a way that the guide spindle (12) is rotated about its longitudinal axis when being axially displaced such that a torque is exerted on the form-fitting element (2,6) that interacts with the projections and/or recesses of the guide torque (12).

5. The orthopaedic device according to one of the preceding claims, **characterised in that** the first form-fitting element (2) and/or the second form-fitting element (6) comprises at least two partial form-fitting elements (22) which can be moved independently of one another in the axial direction.

6. The orthopaedic device according to claim 5, **characterised in that** the at least two partial form-fitting elements (22) comprise the same projections and/or recesses, which are at an offset to each other in the circumferential direction.

7. The orthopaedic device according to claim 5 or 6, **characterised in that** the at least two partial form-fitting elements (22) are spaced apart from each other in the axial direction when the first form-fitting element (2) and the second form-fitting element (6) are not engaged.

8. The orthopaedic device according to one of the preceding claims, **characterised in that** the first joint element is an upper body element and the second joint element is an upper leg element and that the device comprises a pelvic element, wherein the two form-fitting elements (2,6) can be engaged and disengaged by moving the upper body element relative to the pelvic element.

9. The orthopaedic device according to one of the claims 2 to 8, **characterised in that** at least some, preferably all projections of the two form-fitting elements (2,6) comprise undercut toothing.

10. The orthopaedic device according to one of the preceding claims, **characterised in that** the form-fitting elements (2,6) can be engaged with each other by moving one of the form-fitting elements (2,6) towards the other form-fitting element (2,6), which is mounted such that it can be rotated relative to the component on which it is arranged.

## Revendications

1. Dispositif orthopédique comprenant
une articulation qui comprend
- un premier élément d'articulation, qui présente
• un premier bras d'articulation muni d'un premier élément de liaison par complémentarité de forme (2), et
- un deuxième élément d'articulation pouvant pivoter par rapport au premier élément d'articulation, qui présente
• un deuxième bras d'articulation et
• un levier d'application de force muni d'un deuxième élément de liaison par complémentarité de forme, et
• un accumulateur d'énergie mécanique disposé entre le levier d'application de force et le deuxième bras d'articulation,
l'accumulateur d'énergie mécanique pouvant être chargé et déchargé par un pivotement du premier bras d'articulation par rapport au deuxième bras d'articulation lorsque le premier élément de liaison par complémentarité de forme (2) est en prise avec le deuxième élément de liaison par complémentarité de forme (6),
**caractérisé en ce que** le dispositif comprend un dispositif de blocage assurent que, indépendamment de la position du premier élément de liaison par complémentarité de forme (2) et du deuxième élément de liaison par complémentarité de forme (6) l'un par rapport à l'autre, les deux éléments de liaison par complémentarité de forme (2, 6) peuvent être mis en prise de telle sorte qu'une force agissant par l'accumulateur d'énergie mécanique chargé est transmise du deuxième élément de liaison par complémentarité de forme (6) au premier élément de liaison par complémentarité de forme (2).

2. Dispositif orthopédique selon la revendication 1,
**caractérisé en ce que** le dispositif de blocage est conçu pour faire tourner les éléments de liaison par complémentarité de forme (2, 6) l'un par rapport à l'autre lors de la mise en prise ou après la mise en prise des deux éléments de liaison par complémentarité de forme (2, 6).

3. Dispositif orthopédique selon la revendication 2,
**caractérisé en ce que** le premier de liaison par complémentarité de forme (2) et le deuxième élément de liaison par complémentarité de forme (6) présentent des saillies et/ou des cavités frontales, et le dispositif de blocage présente une broche de guidage (12) faisant saillie axialement de l'un des éléments de liaison par complémentarité de forme (2, 6), laquelle présente des saillies et/ou des cavités et est conçue pour coopérer avec l'autre élément de liaison par complémentarité de forme (6, 2) respectif.

4. Dispositif orthopédique selon la revendication 3,
**caractérisé en ce que** la broche de guidage (12) peut être déplacée dans la direction axiale par rapport à l'élément de liaison par complémentarité de forme (2, 6) duquel elle fait saillie axialement, la broche de guidage (12) étant réalisée de telle sorte que, lors du déplacement axial, la broche de guidage (12) est tournée autour de son axe longitudinal, de sorte qu'un couple de rotation est exercé sur l'élément de liaison par complémentarité de forme (2, 6) qui coopère avec les saillies et/ou les cavités de la broche de guidage (12).

5. Dispositif orthopédique selon l'une des revendications précédentes, **caractérisé en ce que** le premier élément de liaison par complémentarité de forme (2) et/ou le deuxième élément de liaison par complémentarité de forme (6) comporte au moins deux éléments de liaison par complémentarité de forme partielle (22) qui sont mobiles dans la direction axiale indépendamment l'un de l'autre.

6. Dispositif orthopédique selon la revendication 5,
**caractérisé en ce que** lesdits au moins deux éléments de liaison par complémentarité de forme partielle (22) présentent les mêmes saillies et/ou cavités qui sont décalées les unes par rapport aux autres dans la direction circonférentielle.

7. Dispositif orthopédique selon la revendication 5 ou 6,
**caractérisé en ce que** lesdits au moins deux éléments de liaison par complémentarité de forme partielle (22) sont espacés l'un de l'autre dans la direction axiale lorsque le premier élément de de liaison par complémentarité de forme (2) et le deuxième élément de liaison par complémentarité de forme (6) ne sont pas en prise l'un avec l'autre.

8. Dispositif orthopédique selon l'une des revendications précédentes, **caractérisé en ce que** le premier élément d'articulation est un élément de torse et le deuxième élément d'articulation est un élément de cuisse, et le dispositif comprend un élément de bassin, les deux éléments de liaison par complémentarité de forme (2, 6) pouvant être mis en prise et hors prise par un déplacement de l'élément de torse par rapport à l'élément de bassin.

9. Dispositif orthopédique selon l'une des revendications 2 à 8,
**caractérisé en ce qu'**au moins certaines, de préférence toutes les saillies des deux éléments de liaison par complémentarité de forme (2, 6) présentent une denture en contre-dépouille.

10. Dispositif orthopédique selon l'une des revendications précédentes, **caractérisé en ce que** les éléments de liaison par complémentarité de forme (2, 6) peuvent être mis en prise l'un avec l'autre par un déplacement de l'un des éléments de liaison par complémentarité de forme (2, 6) vers l'autre élément de liaison par complémentarité de forme (2, 6) qui est monté rotatif dans une direction par rapport au composant sur lequel il est agencé.
